# EUROPÄISCHE PATENTSCHRIFT

(11) **EP 2 987 519 B1**
(45) Veröffentlichungstag und Bekanntmachung des Hinweises auf die Patenterteilung: **21.10.2020**
(21) Anmeldenummer: 15181206.2
(22) Anmeldetag: 17.08.2015
(51) Int. Cl.: A61M 5/32, A61M 5/42, A61M 5/46

(54) **SUBKUTANINJEKTIONSNADEL**
HYPODERMIC NEEDLE
AIGUILLE D'INJECTION SOUS-CUTANEE

(30) Priorität: 19.08.2014 DE 102014111785
(43) Veröffentlichungstag der Anmeldung: 24.02.2016
(73) Patentinhaber: IME-DC GmbH International Medical Equipment - Diabetes Care, 95030 Hof (DE)
(72) Erfinder: Kamalak, Serif, 95032 Hof (DE)
(74) Vertreter: Sperschneider, Alexandra

(56) Entgegenhaltungen:
- WO-A1-2011/144604
- WO-A1-2015/071390
- WO-A2-2012/092343
- BE-A1- 378 924
- US-A1- 2004 092 875

## Beschreibung

Die Erfindung betrifft eine Subkutaninjektionsnadel, aufweisend eine Hohlnadel, die in einer Nadelnabe beidseitig vorstehend fixiert ist, wobei das eine Ende mit der Nadelnabe aus der Stirnwand einer auf ein Verbindungsstück einer Spritze aufschraubbaren oder aufsteckbaren Muffe vorsteht, die das andere Ende der Hohlnadel mindestens teilweise umgibt, und dass das in der Muffe befindliche Ende bei der Fixierung der Muffe an dem Verbindungsstück eine Gummimembran der Spritze durchstößt, die zumindest einen Teil der Endfläche des Verbindungsstückes bildet, wobei bei Gebrauch der Subkutaninjektionsnadel die Stirnfläche die Anlagefläche der Nadelnabe ist und an der Haut anliegt.

Subkutaninjektionsnadeln der gattungsgemäßen Art werden in der Medizin dazu benutzt, in menschliches oder tierisches Gewebe einzudringen, um mit Hilfe einer Spritze Flüssigkeiten zu injizieren oder zu entnehmen. Besonders feine Hohlnadeln bzw. Kanülen werden z. B. für die Injektion von Insulin verwendet. Den Durchmessern dieser Hohlnadeln sind jeweils farbliche Kennzeichnungen zugeordnet. Entsprechend sind die Muffen, die aus Kunststoff bestehen, gefärbt. Die Spitzen der Hohlnadeln können verschieden ausgeführte Schliffe aufweisen, die jeweils von der Anwendung abhängig sind.

Diabetiker setzen sich Insulinspritzen in der Regel in Eigeninjektion. Für die Eigeninjektion werden in der Regel Injektionsgeräte verwendet, die als vorgefüllte Injektionsgeräte oder als nachfüllbare Injektionsgeräte ausgeführt sind. Solche Geräte werden auch als Pen bezeichnet und weisen ein Verbindungsstück an der Injektionsflüssigkeitspatrone, z.B. Insulinpatrone, auf, um daran eine Subkutaninjektionsnadel anschrauben oder aufrasten zu können. Solche Verbindungsstücke können aber auch Bestandteil des Gerätes selbst sein, ebenso Bestandteil einer Spritze. Dies ist erforderlich, da nach jeder Injektion die Nadel gewechselt werden sollte. Solche Injektionsgeräte lösen vermehrt die an sich bekannten Spritzen ab, die in der Regel ebenfalls auswechselbare Injektionsnadeln, also Hohlnadeln, aufweisen bzw. mit solchen bestückt werden können. Derartige Pens weisen Dosierungsstellglieder auf, mit welchen die Abgabemenge des Insulins aus der eingesetzten Ampulle bestimmt werden kann. Die austauschbaren Subkutaninjektionsnadeln weisen eine gattungsgemäße Ausbildung auf. An den Innenflächen der Muffe der Nadeleinheit kann ein Innengewinde vorgesehen sein, das auf das Außengewinde des Verbindungsstückes des Injektionsgerätes oder der Spritze aufschraubbar ist. Bei anderen Ausführungen ist vorgesehen, dass die Muffe mit Rastelementen versehen ist, die beispielsweise in Nuten an dem Verbindungsstück einrasten, so dass die Muffe über den Verbindungsaufsatz aufschiebbar und daran mittels dieser Rastmittel gehalten wird. Subkutaninjektionsnadeln dieser Art sind beispielsweise in der DE 695 24 867 T2 (EP 0 750 518 B1) beschrieben. Damit die Nadel geschützt ist, wird über die Muffe eine Schutzkappe aufgeschoben, die die Muffe rückseitig übersteht, so dass mittels eines Versiegelungsblättchens oder einer Folie die Öffnung steril versiegelt werden kann, um die Nadel steril verpackt vorhalten zu können. Auch nach Gebrauch kann die Schutzkappe aufgesteckt und mit ihr von dem Verbindungsstück der Spritze abgeschraubt oder abgezogen werden.

Subkutaninjektionsnadeln der gattungsgemäßen Art sind ferner aus der WO 2011/144604 A1, der US 2004/092875 A1, der WO 2012/092343 A2, der BE 378 924 A1, der DE 695 24 254 T2 (EP 0 799 065 B1) und der DE 601 31 847 T2 (EP 1 289 586 B1) bekannt.

Bei allen bekannten Subkutaninjektionsnadeln ist die Nadelnabe kleinflächig ausgebildet. Sie weist in der Regel einen Durchmesser von lediglich 3 mm auf und ist bei vielen Ausführungen kegelstumpfförmig an der Spitze ausgebildet und umschließt die Hohlnadel vollständig. Die bei Verabreichen der Spritze an der Haut anliegende Fläche ist also sehr klein. Es hat sich gezeigt, dass insbesondere bei der Eigeninjektion die Nadel sehr tief in das Gewebe gedrückt wird und dabei der Kopf der Nadelnabe mit in die Hautöffnung hineingedrückt wird, was zu erhöhter Schmerzempfindung des Patienten führen kann. Des Weiteren dringt durch diese Auslegung die Nadelspitze tiefer in das Gewebe ein, als es eigentlich für die Verabreichung des Injektionsmittels erforderlich ist, wobei hier "Injektionsmittel" nicht nur für Insulin, sondern auch für andere Injektionsflüssigkeiten steht.

Ausgehend von der dargestellten Problematik herkömmlicher Subkutaninjektionsnadeln liegt der Erfindung die Aufgabe zugrunde, diese so auszubilden, dass die Möglichkeit der Schmerzauslösung beim Injizieren der Injektionsflüssigkeit in das Gewebe eines Menschen oder Tieres merklich reduziert wird und die Schmerzauslösung nur durch die Nadel selbst bestimmt ist.

Gelöst wird die Aufgabe durch Ausgestaltung der Subkutaninjektionsnadel gemäß der im Anspruch 1 angegebenen Lehre. Demnach ist unter Anderem, die an der Haut anliegende Stirnfläche der Nadelnabe mindestens 6 mm² groß. Versuche haben gezeigt, dass nur dann, wenn die Fläche der Stirnseite der Nadelnabe ausreichend groß ist, die Nadel selbst in der Eindringtiefe im Gewebe durch ihre Länge bestimmt ist, die beispielsweise 6 mm, 8 mm 10 mm oder 16mm bei einem Durchmesser von 0,2 mm, 0,3mm, 0,4 mm, 0,8 mm betragen kann. Die Stirnfläche der Nadelnabe selbst vermag ab der angegebenen Größe keine zusätzliche Schmerzen auszulösen und begrenzt darüber hinaus auch die Eindringtiefe der Nadel optimal.

Je größer die Anlagefläche ist, desto geringer ist das Risiko der zusätzlichen Schmerzauslösung. Wenn die Anlagefläche ≥ 10 mm² ist, verteilt sich der Druck auf eine noch größere Anlagefläche, so dass einzelne Nervenenden durch einen geringeren Druck gereizt werden. Als optimal haben sich Anlageflächen zwischen 30 mm² und 80 mm² für Subkutaninjektionsnadeln mit üblichen Durchmessern von 0,16 mm bis 0,5 mm erwiesen, die zur Verabreichung von Insulin bei Diabetikern zum Einsatz kommen. Vorzugsweise sollte die Fläche zwischen 30 mm² bis 70 mm² groß sein, damit der Außendurchmesser der Nadelnabe nicht den Außendurchmesser der Muffe übersteigt und ein Ringflansch an der Muffe, die z.B. einen Durchmesser von 8 mm bis 10 mm aufweisen kann, noch gegeben ist, auf den eine Schutzkappe aufsetzbar ist, die von der Nadelnabe gehalten wird. Hierbei handelt es sich um Rast- oder Klemmverbindungen bekannter Art, die zur Anwendung kommen können. Darüber hinaus kann über diese Anordnung ein Aufbewahrungsbehälter in Form einer weiteren Schutzkappe aufgesteckt werden, die die Muffe rückseitig geringfügig übersteht und in der Regel einen Rand aufweist, so dass eine Versiegelungsfolie auf die Öffnung aufgebracht werden kann, wodurch eine sterile Verpackung der Subkutaninjektionsnadel möglich ist.

Grundsätzlich kann die Nadelnabe eine runde oder auch eine polygonale Querschnittsform aufweisen. Entsprechend müssen dann die Schutzkappen ausgebildet sein, um die Nadel gegen Abbiegen und Verschmutzung zu schützen. Das Gleiche gilt auch für die Muffe. Im Falle, dass es sich um eine Steckmuffe handelt, kann diese auch mit polygonalem Querschnitt, z. B. quadratisch, ausgeführt sein. Bevorzugt ist aber die runde Form, da neben dem Aufrasten dann auch ein Aufschrauben durch ein eingebrachtes Innengewinde auf ein Außengewinde des Verbindungsstückes des Injektionsgerätes, der eingesetzten Injektionsflüssigkeitspatrone oder der Spritze bzw. einer Ampulle möglich ist. Die Muffe und die Nabe sowie die Schutzkappe sind aus Kunststoff gefertigt; die Nadel aus Edelstahl.

Die Nadelnabe kann darüber hinaus eine gebogene Anlagefläche und selbst eine definierte Überstandslänge aufweisen. Beispielsweise kann die Hohlnadel 6 mm, 8 mm oder 10 mm aus der Anlagefläche vorstehen. Die Fläche kann dabei in gebogener Ausführung konvex gebogen oder auch wellenförmig ausgeführt sein, wobei die Hohlnadel stets zentrisch angeordnet ist. Der Durchmesser der Nadelnabe im Bereich der Anlagefläche kann bei zylindrischer Ausführung gleich dem Durchmesser an der Stirnwand der Muffe sein. Er kann aber auch etwas größer ausgeführt sein, so dass eine trapezförmige Mantelwand gegeben ist, während bei der ersten Ausführung eine geradlinige Mantelwand des zylinderförmigen Ansatzes gegeben ist. Bei der trapezförmig sich nach außen öffnenden Ausführung können ringförmige Nuten in die Mantelfläche eingearbeitet sein, um das Einrasten einer Schutzkappe zu ermöglichen. Derartige Nuten können auch bei der zylinderförmigen Ausführung vorgesehen sein, wenn auf ein Innengewinde in der Muffe verzichtet wird bzw. eine Aufsteckausführung gewählt wird.

Die Anlagefläche der Nadelnabe sollte in einem Winkel von 90° zur Längsachse verlaufend vorgesehen sein. Sie kann eine konvexe Wölbung aufweisen. Im Falle der konvexen Wölbung, also der zum Mittelpunkt hin überstehenden kuppenartigen Auslegung, ist zwar ein leichtes Eindrücken der Oberfläche in die Haut und/oder das Gewebe gegeben. Die Druckverteilung verläuft aber ebenfalls optimal, so dass keine zusätzliche Schmerzauslösung befürchtet werden muss. Wenn eine , nicht erfindungsgemäße , Krümmung, also in Nadelnähe eine niedrigere Anordnung gegenüber dem Rand gegeben ist, schiebt sich die Anlagefläche über das Gewebe und drückt dieses in den Hohlraum hinein.

Versuche haben gezeigt, dass eine angenehme Druckverteilung gegeben ist, wenn die Oberfläche der Anlagefläche mit Noppen besetzt oder aufgeraut ist oder ein definiertes Profilmuster aufweist, so dass eine Vielzahl von Anlagepunkten an der Haut gegeben ist, während die Nadelspitze in das Gewebe eindringt bzw. eingedrungen ist.

Die erfindungsgemäßen Ausbildungen sind in den Unteransprüchen im Detail angegeben.

Die Erfindung wird nachfolgend anhand der in den Zeichnungen dargestellten Ausführungsbeispiele ergänzend erläutert.

In den Zeichnungen zeigen:
- FIG 1: eine bekannte Subkutaninjektionsnadel mit einer Schutzkappe und einem Aufbewahrungsbehälter in einer Explosionsdarstellung,
- FIG 2: in einer Explosionsdarstellung eine Subkutaninjektionsnadel nach der Erfindung mit einer Schutzkappe und einem Aufbewahrungsbehälter sowie einer perspektivischen Darstellung mit glatter Anlagefläche und zylinderförmiger Nadelnabe,
- FIG 3: eine Abwandlung zu dem Ausführungsbeispiel in FIG 2 mit einer auf dem Kopf stehenden trapezförmigen Ausführung der Nadelnabe,
- FIG 4: eine erfindungsgemäß ausgebildete Subkutaninjektionsnadel mit einer zylinderförmigen Nadelnabe mit einer strukturiert aufgerauten Anlagefläche,
- FIG 5: ein Beispiel einer Abwandlung zu FIG 3, bei der die Anlagefläche ebenfalls aufgeraut strukturiert ist,
- FIG 6: eine in einer Explosionszeichnung dargestellte Subkutaninjektionsnadel mit einer konkav ausgebildeten Anlagefläche mit trapezförmiger Ausbildung der Mantelfläche der Nadelnabe,
- FIG 7: eine Subkutaninjektionsnadel mit einer trapezförmig ausgebildeten Nadelnabe mit konvex verlaufender Anlagefläche,
- FIG 8: ein Ausführungsbeispiel einer zylindrischen Nadelnabe mit einer konkaven Anlagefläche und
- FIG 9: eine solche zylinderförmige Nadelnabe mit einer konvexen Anlagefläche.

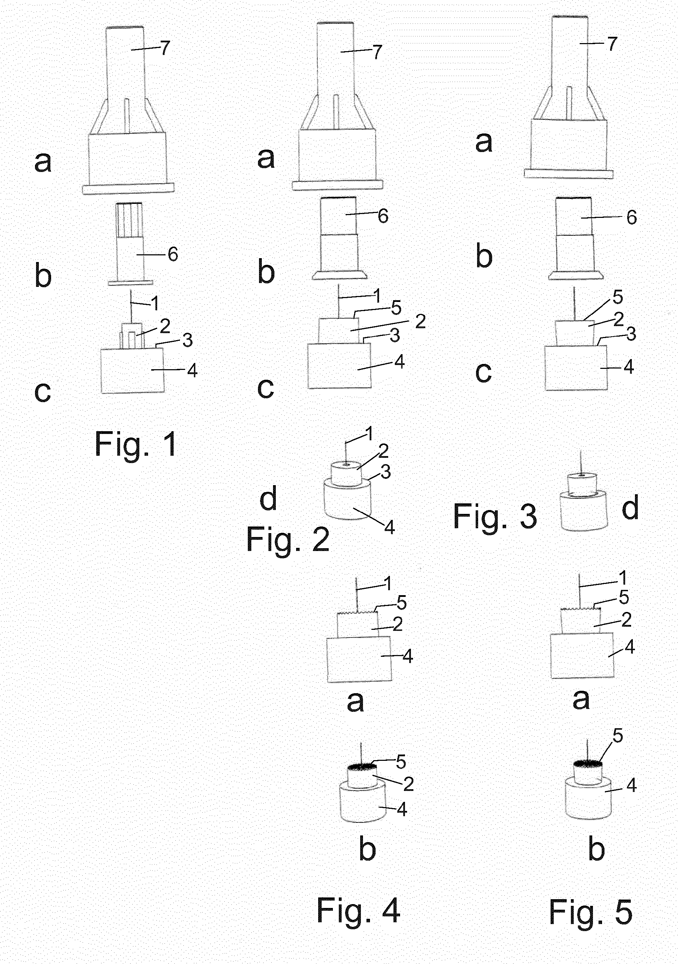

In FIG 1 ist eine bekannte Subkutaninjektionsnadel dargestellt. Diese weist eine Hohlnadel 1 auf, die in einer Nadelnabe 2 befestigt ist bzw. von einer solchen Nabelnabe, die aus Kunststoff besteht, umspritzt ist. Die Nadelnabe 2 ihrerseits ist an eine Muffe 4 angeformt. Dabei kann es sich um eine Schraub- oder Aufsteckmuffe handeln oder um eine kombinierte Muffe, die sowohl schraubbar als auch aufsteckbar ist. Die Muffe weist zum Aufschrauben ein Innengewinde auf. Sie ist, wie die Nadelnabe, ebenfalls rund ausgebildet und weist beispielsweise einen Au-ßendurchmesser von 10 mm bis 12 mm auf, während der Durchmesser der runden Nadelnabe maximal 3 mm beträgt. Auf der Nadelnabe 2 sind ferner Längsrippen aufgebracht, um klemmend hierauf eine Schutzkappe 6 aufstecken zu können, die mit einem Ringflansch auf die Stirnfläche 3 der Stirnwand der Muffe aufgreift. Durch die Schutzkappe 6 ist die Hohlnadel 1, die aus der Nadelnabe 2 um ein definiertes Maß, z. B. 6 mm oder 8mm, vorsteht, bei Nichtgebrauch geschützt. Zusätzlich ist ein Aufbewahrungsbehälter 7 vorgesehen, der über die Schutzkappe 6 und die Muffe 4 geschoben werden kann und das Gebinde hermetisch abschließt. Der untere Flansch des Aufbewahrungsbehälters 7 übersteht den unteren Rand der Muffe 4 und ist mit einer Versiegelungsfolie verschweißt, so dass in dieser zusammengesetzten Form die Subkutaninjektionsnadel steril verpackt ist und nach Entfernen der Versiegelungsfolie auf ein Verbindungsstück einer Spritze bzw. eines Injektionsgerätes aufschraubbar ist, wobei das nicht sichtbare, konzentrisch in der Muffe 4 angeordnete Hohlnadelende eine Gummimembran des Injektionsgerätes durchstößt. Aus der Abbildung ist ferner ersichtlich, dass die Nadelnabe 2 einen relativ kleinen Durchmesser aufweist, nämlich ≤ 3 mm ist. Teilweise sind kegelspitzförmige Spitzen noch angeformt. Bei der Benutzung einer solchen Subkutaninjektionsnadel, zusammen mit einer Spritze oder einem Injektionsgerät, ist das Eindringen des Kopfes der Nadelnabe 2 in die durch die Hohlnadel 1 entstehende Gewebeöffnung nicht auszuschließen, so dass in diesem Bereich ein höheres Schmerzempfinden des Patienten gegeben ist.

Erfindungsgemäß ist nun, wie aus FIG 2c und 2d ersichtlich, die Anlagefläche 5 der Nadelnabe 2, und damit der Durchmesser der Nadelnabe, wesentlich vergrößert. Der Durchmesser ist so groß gewählt, dass die Fläche z. B. ≥ 10 mm² beträgt, vorzugweise 30 mm² bis 70 mm², so dass eine große Anlagefläche an der Haut gegeben ist und die Nadel mit einem verteilten Flächendruck an der Haut anliegt, wodurch nicht nur die Eindringtiefe der Hohlnadel 1 selbst in das Gewebe begrenzt ist, sondern auch die Nervenzellenenden nicht einem hohen schmerzauslösenden Druck unterworfen sind. Im Übrigen ist der Durchmesser der Nadelnabe 2 so gewählt, dass er kleiner ist als der Durchmesser der Muffe, so dass auf dem verbleibenden Ringflansch der Stirnfläche 3 der Muffe 4 der Ringflansch an der aufsetzbaren Schutzkappe 6 in bekannter Weise aufgesetzt und diese noch von einem Aufbewahrungsbehälter 7 umgeben werden kann, damit eine sterile Verpackung derselben, wie zuvor anhand von FIG 1 beschrieben, vorgenommen werden kann. Im Ausführungsbeispiel gemäß FIG 2c und 2d in der perspektivischen Darstellung ist ersichtlich, dass nicht nur die Muffe Zylinderform besitzt, sondern auch die Nadelnabe 2.

Im Gegensatz dazu ist in FIG 3 eine sich nach oben öffnende, trapezförmige Ausbildung der Nadelnabe 2 vorgesehen. Das bedeutet, dass der Durchmesser der Nadelnabe 2 im Bereich der Oberfläche der Stirnwand der Muffe 4 kleiner ist als der an der Anlagefläche 5. Hierdurch ist es möglich, Schutzhauben 6 aufzustecken, die elastisch federnde Wände aufweisen und selbsthaltend auf die Nadelnabe 2 aufrasten können. Dieses Gebilde kann sodann von dem Aufbewahrungsbehälter 7 geschützt werden, der, wie anhand FIG 1 beschrieben, aufsetzbar und versiegelbar ist. In FIG 3d ist in einer perspektivischen Darstellung die Subkutaninjektionsnadel mit der trapezförmigen Nadelnabe 2 dargestellt.

In FIG 4 ist eine Abwandlung des Ausführungsbeispiels gemäß FIG 2 dargestellt. Es ist aus FIG 4a und FIG 4b ersichtlich, dass die Anlagefläche 5 der Nadelnabe 2 eine aufgeraute Fläche, z. B. eine Riffelung, aufweist. Es hat sich gezeigt, dass es als angenehm empfunden wird, wenn eine aufgeraute Anlagefläche an der Haut anliegt. Im Übrigen weist die Nabelnabe 2 Zylinderform auf.

In FIG 5 ist eine Abwandlung zu dem Ausführungsbeispiel in FIG 3 dargestellt. Hier bildet ein auf dem Kopf stehender Kegelstumpf die Nabelnabe 2, in der konzentrisch die Hohlnadel 1 gelagert ist. Auch dort ist die Anlagefläche 5 mit einer Aufrauung, z. B. Riffelung, versehen, wie auch aus der perspektivischen Darstellung gemäß FIG 5b ersichtlich ist. Anstelle einer Riffelung können auch Noppen über die Ablagefläche verteilt vorgesehen sein.

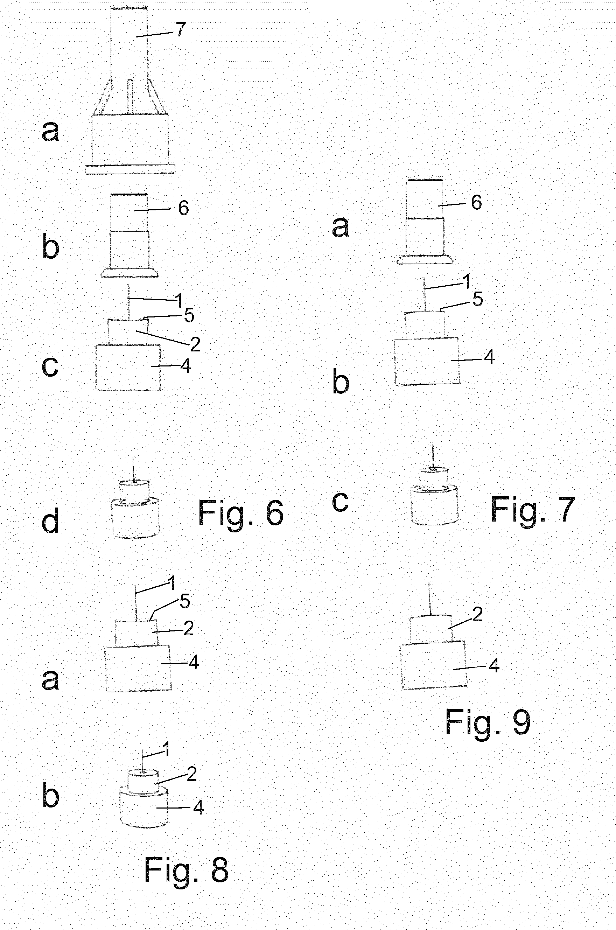

In FIG 6 ist eine Abwandlung des Ausführungsbeispiels nach FIG 5 dargestellt. Das Ausführungsbeispiel unterscheidet sich dadurch, dass die Anlagefläche 5 eine glatte Oberfläche aufweist und konkav gebogen ausgeführt ist. Beim Einführen der Nadel in ein Gewebe wird dieses Gewebe also gewissermaßen in dem Bogen gestaut. Dies führt zu positiver Druckverteilung. FIG 6d verdeutlicht die runde Struktur der Subkutaninjektionsnadel, die im Übrigen mit einer Schutzkappe 6 und mit einem Aufbewahrungsbehälter 7 umgeben werden kann. Die Unterfiguren sind jeweils mit a, b, c und d bezeichnet.

Das Ausführungsbeispiel in FIG 7 zeigt eine weitere Variante zu FIG 6, aus der ersichtlich ist, dass die Anlagefläche 5 auch konvex ausgebildet sein kann, so dass auch hierüber eine größere Druckverteilung bei Anlage der Anlagefläche an der Haut und beim Eindringen der Hohlnadel 1 in das Gewebe gegeben ist. Die FIG 7c zeigt dieselbe Subkutaninjektionsnadel in einer perspektivischen Darstellung, aus der auch ersichtlich ist, dass die Schutzkappe 6 in gleicher Weise aufsetzbar ist wie in den Ausführungsbeispielen zuvor.

FIG 8 zeigt ein weiteres Ausführungsbeispiel zu FIG 4 bzw. FIG 2. Die zylinderförmige Nadelnabe 2 ist obenseitig konkav ausgebildet, weist also eine konkave Anlagefläche auf. Die perspektivischer Darstellung ist in FIG 8b wiedergegeben.

FIG 9 zeigt eine Abwandlung zu der Ausführungsform in FIG 7, und zwar ist auch dort die Nadelnabe 2 zylinderförmig ausgebildet, wie in FIG 2 und FIG 4 dargestellt.

### Bezugszeichenliste

- 1: Hohlnadel
- 2: Nadelnabe
- 3: Stirnwand
- 4: Muffe
- 5: Anlagefläche
- 6: Schutzkappe
- 7: Aufbewahrungsbehälter

## Patentansprüche

1. Subkutaninjektionsnadel, aufweisend eine Hohlnadel (1), die in einer Nadelnabe (2) beidseitig vorstehend fixiert ist, wobei das eine Ende mit der Nadelnabe (2) aus der Stirnwand (3) einer auf ein Verbindungsstück einer Spritze aufschraubbaren oder aufsteckbaren Muffe (4) vorsteht, die das andere Ende der Hohlnadel (1) mindestens teilweise umgibt, und dass das in der Muffe befindliche Ende bei der Fixierung der Muffe (4) an dem Verbindungsstück eine Gummimembran der Spritze durchstößt, die zumindest einen Teil der Endfläche des Verbindungsstückes bildet, wobei bei Gebrauch der Subkutaninjektionsnadel die Stirnfläche die Anlagefläche (5) der Nadelnabe (2) ist und an der Haut anliegt, **dadurch gekennzeichnet, dass** die an der Haut anliegende Anlagefläche (5) der Nadelnabe mindestens 6 mm² groß ist, wobei der Durchmesser der Nadelnabe (2) so gewählt ist, dass er kleiner als der Durchmesser der Muffe (4) ist, und dass die Anlagefläche (5) in gebogener Ausführung oder wellenförmig ausgebildet ist, wobei die Anlagefläche (5) in gebogener Ausführung eine konvexe Form aufweist.

2. Subkutaninj ektionsnadel nach Anspruch 1, **dadurch gekennzeichnet, dass** die Anlagefläche (5) ≥ 10 mm² ist oder eine Größe zwischen 30 mm² und 80 mm² aufweist, vorzugsweise 30 mm² bis 70 mm² groß ist.

3. Subkutaninjektionsnadel nach Anspruch 1 oder 2, **dadurch gekennzeichnet, dass** die Nadelnabe (2) eine runde oder polygonale Querschnittsform und eine gerade oder gebogene Anlagefläche (5) und eine definierte Länge aufweist.

4. Subkutaninjektionsnadel nach Anspruch 3, **dadurch gekennzeichnet, dass** der Durchmesser der Anlagefläche (5) der Nadelnabe (2) gleich dem Durchmesser an der Stirnwand (3) der Muffe (4) ist oder größer und die Mantelwand der Nadelnabe (2) geradlinig oder trapezförmig, nach außen sich öffnend ausgeführt ist und/oder abschnittsweise oder ringförmig Nuten zum Einrasten einer Schutzkappe (6) aufweist.

5. Subkutaninjektionsnadel nach Anspruch 1 oder 3, **dadurch gekennzeichnet, dass** die Anlagefläche (5) im Winkel von 90° zur Nadellängsachse verlaufend ausgebildet ist und eine geradlinige, konvexe, konkave oder eine wellenförmige Struktur aufweist.

6. Subkutaninjektionsnadel nach Anspruch 1 oder 5, **dadurch gekennzeichnet, dass** die Oberfläche der Anlagefläche (5) mit Noppen besetzt ist, aufgeraut ist oder ein definiertes Profilmuster aufweist.

7. Subkutaninj ektionsnadel nach Anspruch 1, **dadurch gekennzeichnet, dass** die Subkutaninj ektionsnadel in bekannter Weise von einem Aufbewahrungsbehälter (7) umgeben ist, der über die Schutzkappe (6) oder die Hohlnadel (1) und die Muffe (4) greift und mit einer Abdeckung an der offenen Muffenseite steril versiegelt ist.

## Claims

1. Hypodermic needle comprising a hollow needle (1), which is fixed protruding on both sides, wherein the one end with the needle hub (2) protrudes from the end wall (3) of a sleeve (4) which can be screwed or plugged onto a connecting piece of a syringe, which at least partially surrounds the other end of the hollow needle (1), and that the end inside the sleeve penetrates a rubber membrane of the syringe when fixing the sleeve (4) to the connecting piece, which forms at least a part of the end face of the connecting piece, wherein during the use of the hypodermic needle the front surface is the contact surface (5) of the needle hub (2) and is in contact with the skin, **characterized in that** the contact surface (5) of the needle hub touching the skin is at least 6 mm² in size, wherein the diameter of the needle hub (2) is selected so that it is smaller than the diameter of the sleeve (4), and that the contact surface (5) is formed in a curved design or wave-shaped, wherein the contact surface (5) in curved design has a convex shape.

2. Hypodermic needle according to claim 1,
**characterized in that** the contact surface (5) is ≥ 10 mm² or has a size between 30 mm² and 80 mm², is preferably 30 mm² to 70 mm² in size.

3. Hypodermic needle according to claim 1 or 2,
**characterized in that** the needle hub (2) has a round or polygonal cross-section shape and a straight or curved contact surface (5) and a defined length.

4. Hypodermic needle according to claim 3,
**characterized in that** the diameter of the contact surface (5) of the needle hub (2) is equal to or larger than the diameter at the end wall (3) of the sleeve (4) and the shell wall of the needle hub (2) is configured in a straight line or trapezoidal shape, opening outwards, and/or has sectional or annular grooves for snapping a protective cap (6).

5. Hypodermic needle according to claim 1 or 3,
**characterized in that** the contact surface (5) is formed running at an angle of 90° to the longitudinal axis of the needle and has a straight-line, convex, concave or undulating structure.

6. Hypodermic needle according to claim 1 or 5,
**characterized in that** the surface of the contact surface (5) is covered with nubs, is roughened or has a defined profile pattern.

7. Hypodermic needle according to claim 1,
**characterized in that** the hypodermic needle is surrounded in a known manner by a storage container (7), which reaches over the protective cap (6) or the hollow needle (1) and the sleeve (4) and is sterilely sealed with a cover on the open sleeve side.

## Revendications

1. Aiguille d'injection sous-cutanée, présentant une aiguille creuse (1), qui est fixée dans un moyeu d'aiguille (2) de manière à faire saillie des deux côtés, dans laquelle l'une des extrémités fait saillie avec le moyeu d'aiguille (2) de la paroi frontale (3) d'un manchon (4), pouvant être vissé ou emboîté sur une pièce de liaison d'une seringue, qui entoure au moins en partie l'autre extrémité de l'aiguille creuse (1), et que l'extrémité se trouvant dans le manchon, lors de la fixation du manchon (4) sur la pièce de liaison, transperce une membrane en caoutchouc de la seringue, qui forme au moins une partie de la surface d'extrémité de la pièce de liaison, dans laquelle lors de l'utilisation de l'aiguille d'injection sous-cutanée, la surface frontale est la surface d'appui (5) du moyeu d'aiguille (2) et s'applique sur la peau, **caractérisée en ce que** la surface d'appui (5) du moyeu d'aiguille s'appliquant sur la peau mesure au moins 6 mm², dans laquelle le diamètre du moyeu d'aiguille (2) est choisi de sorte qu'il est plus petit que le diamètre du manchon (4), et que la surface d'appui (5) est réalisée de manière courbée ou ondulée, dans laquelle la surface d'appui (5), dans la réalisation courbée, présente une forme convexe.

2. Aiguille d'injection sous-cutanée selon la revendication 1, **caractérisée en ce que** la surface d'appui (5) ≥ 10 mm² ou présente une dimension comprise entre 30 mm² et 80 mm², de préférence mesure 30 mm² à 70 mm².

3. Aiguille d'injection sous-cutanée selon la revendication 1 ou 2, **caractérisée en ce que** le moyeu d'aiguille (2) présente une forme en section transversale ronde ou polygonale et une surface d'appui (5) rectiligne ou courbée et une longueur définie.

4. Aiguille d'injection sous-cutanée selon la revendication 3, **caractérisée en ce que** le diamètre de la surface d'appui (5) du moyeu d'aiguille (2) est supérieur ou égal au diamètre sur la paroi frontale (3) du manchon (4) et la paroi d'enveloppe du moyeu d'aiguille (2) est rectiligne ou trapézoïdale, de manière à s'ouvrir vers l'extérieur et/ou présente sur certaines parties ou de manière annulaire des rainures pour l'encliquetage d'un capuchon de protection (6).

5. Aiguille d'injection sous-cutanée selon la revendication 1 ou 3, **caractérisée en ce que** la surface d'appui (5) est réalisée de manière à s'étendre par rapport à l'axe longitudinal d'aiguille selon un angle de 90° et présente une structure rectiligne, convexe, concave ou ondulée.

6. Aiguille d'injection sous-cutanée selon la revendication 1 ou 5, **caractérisée en ce que** la surface de la surface d'appui (5) est garnie de picots, rendue rugueuse ou présente un motif profilé défini.

7. Aiguille d'injection sous-cutanée selon la revendication 1, **caractérisée en ce que** l'aiguille d'injection sous-cutanée est entourée de manière connue par un contenant de stockage (7), qui chevauche le capuchon de protection (6) ou l'aiguille creuse (1) et le manchon (4) et est scellé de manière stérile avec un cache au niveau de la face de manchon ouverte.
